# EUROPEAN PATENT SPECIFICATION

(11) **EP 1 523 991 B1**
(45) Date of publication and mention of the grant of the patent: **18.12.2013**
(21) Application number: 04105134.3
(22) Date of filing: 18.10.2004
(51) Int. Cl.: A61K 39/00, A61P 35/00

(54) **Anti-cancer vaccine**
Impfstoff gegen Krebs
Vaccin contre le cancer

(30) Priority: 18.10.2003 EP 03023500
(43) Date of publication of application: 20.04.2005
(73) Proprietor: Alind & Kraja SHA, Tirana (AL)
(72) Inventor: Kraja, Shualp J., Shkoder (AL)
(74) Representative: von Kreisler Selting Werner

(56) References cited:
- EP-A- 1 111 039
- WO-A-99/38954
- WO-A-03/035104
- US-A- 5 484 596
- OHNO TADAO: "Autologous cancer vaccine: A novel formulation." MICROBIOLOGY AND IMMUNOLOGY, vol. 47, no. 4, 2003, pages 255-263, XP008043136 ISSN: 0385-5600
- RIZZO S, SILVOTTI M. G.: "Potentiated Autologous Tumor Cell and Peripheral Blood Lymphocyte Lysate Vaccination" MOLECULAR BIOTECHNOLOGY, vol. 25, no. 1, 2003, pages 31-35, XP008030560
- KRATZ F ET AL: "Serum proteins as drug carriers of anticancer agents: A review" DRUG DELIVERY, ACADEMIC PRESS, ORLANDO, FL, US, vol. 5, no. 4, 1998, pages 281-299, XP002125686 ISSN: 1071-7544
- HORVATH L ET AL: "Intracranial murine glioma 261 model to study the antitumor activity of autologous cancer vaccines, producing various cytokines" EUROPEAN JOURNAL OF CANCER, PERGAMON PRESS, OXFORD, GB, vol. 33, June 1997 (1997-06), page S24, XP004282696 ISSN: 0959-8049

## Description

The present invention relates to a human anti cancer vaccine, a process for the preparation of said vaccine and its use for preparing a medicament for the treatment or prophylaxis of cancer.

### Background

In the past the treatment of cancer is dominated by chemo- or radio-therapy with all the side effects commonly known to public. Thus, there is an urgent need for new and successful treatment of cancer.

In healthy persons, lymphocytes represent normally ca. 30% of the leukocytes, but in cancerous patients, especially patients suffering from leukemia and lymphoid cancer forms, the percentage of lymphocytes reaches 80-90%, in case of lym phoblastic cancer forms even 90-95% .

Approximately 70-80% of the lymphocytes in peripheral blood and over 90% in the lymphatic liquid from Ductus thoracicus have the characteristic features of thymolymphocyte cells (T). Most of the T lymphocytes have a lifetime of 4-6 years, some T cells 20 years and more. The latter is of great importance for the duration and permanence of an immune defense triggered by a vaccine. The focus of the present invention is on such T lymphocytes.

The maturation of T lymphocyte cells is under the control of 2 genetic alleles: Thy 1 -1 and Thy 1 -2, situated in a locus on the chromosome 9.

The interaction of an antigen with T cell receptor results in T cell activation. The activated cells are specifically operating cells usually called "Tk" (killer cells) and have an im portant rule in cellular immune reaction. Tk cells have the capability to destroy specific antigens (when they have learned to identify them) or the corresponding antigen carrier cells. T lymphocytes of all organisms are able to to destroy specific antigens (when they have learned to identify them) or the corresponding antigen carrier cells. T lymphocytes of all organisms are able to diffuse into the thymodependent zones of lymph nodes by means of lymphatic, venous and arterial circulation. Thus, T lymphocytes do not diffuse only to lymphoid peripheral structures, but also to tissues of non-lymphoid organs. The interactions between those organs is probably mediated by the surface of vascular endothelial cells. This interaction is a mechanism of control in immune surveillance via interaction between T lymphocytes and the presented antigens.

Up to now there are known only a few cancer vaccines based on cancer cells (Ohno, A., Microbiol. Immunol. 47: 255-263 (2003); WO 03/035104). These vaccines are strictly autologous and prepared by fixation of whole cancer cells.

### Summary of the Invention

A new anti cancer vaccine according to claim 1 has been found which does not entail the drawbacks of chemo- and radiotherapy and which has been established by specifically modifying blood components and/or cancer tissue material of cancer patients. The invention provides
(1) a human anti cancer vaccine comprising an anatoxin component which is obtainable by modifying *in vitro* blood serum or the supernatant of homogenized cancer tissue cells of a patient suffering from cancer, or the combined blood serum or the supernatant of homogenized cancer tissue cells of a group of patients with blood serotype O⁺ suffering from cancer, with protein crosslinking agents, preferably formaldehyde;
(2) the vaccine of embodiment (1) above, which additionally comprises a lymphocyte component obtainable by contacting vital lymphocytes of one or several person(s) not suffering from cancer with vital lymphocytes of one or several patient(s) suffering from cancer ("cancer lymphocytes") or with DNA fragments isolated from cancer lymphocytes;
(3) a process for production of a human anticancer vaccine as defined in (1) or (2) above, preferably as defined in embodiment (2) which comprises admixing the anatoxin component with the lymphocyte component;
(4) the use of a vaccine component as defined in (1) or (2) above for preparing a medicament for treatment or prophylaxis of all kinds of cancer in a patient.

### Detailed Description of the Invention

The term "protein crosslinking agent" in the context of present invention means standard textbook reagents used for crosslinking of proteins and peptides (see, alkyl compounds containing at least two functional groups selected from aldehydes, epoxides, reactive esters, isothiocyanates, anhydrides, carbodiimides, preferably aldehydes. Most preferred is formaldehyde, which is preferably used as an aqueous solution.

"Anatoxin" usually designates a material resulting from the treatment of a toxin in such a way that the toxic properties are inactivated whilst the antigenic potency remains intact. In the context of present invention this term means the resulting product after treatment of blood serum or cancer tissue material with a protein crosslinking agent according to the invention, especially if used as the obligatory component of the cancer vaccine.

Thus, in a preferred aspect of embodiment (1) the human anti cancer vaccine is prepared by a process comprising the treatment of blood serum of a patient suffering from cancer with formaldehyde. The kind of cancer the patient is suffering from is arbitrary. Alternatively, the combined blood serum of a group of patients suffering from same or different kinds of cancer can be used. In the latter case, the blood serotype must be 0+ in order to avoid negative reactions to the vaccine due to serological incompatibility.

A preferred aspect of embodiments (1), (2) and (4) is that the patient(s) treated and/or the patient(s) providing the blood or tissue for preparation of the vaccine has/have not been treated by chemo- or radiotherapy before.

Furthermore, in embodiment (1) the final concentration range of formaldehyde used for the treatment of serum is 0.05-0.5% (w/w), preferably 0.1 -0.2% (w/w), most preferably 0.14% in the treatment mixture. In other words, if a 37% (w/w) aqueous formaldehyde solution (generally designated as "formalin") is added to the serum, the added volume is most preferably 4 ml/l.

The incubation time after treatment of the serum with a crosslinking agent according to embodiment (1) is 25-35 days, preferably at least 28 days. The incubation takes place at a temperature of 38 ±2°C, preferably at least 37°C, most preferably at 38-39 °C.

One specific anatoxin component of embodiment (1 ) is obtainable by a process containing the following steps:
(a) extracorporeally coagulating blood of patient(s) suffering from cancer,
(b) centrifuging said blood,
(c) combining the supernatant serum with an aqueous solution of formaldehyde,
(d) incubating the mixture of step (c) at a temperature of 38°C +/- 2°C,
(e) treating the product of step (d) with an aqueous solution of phosphoric acid, preferably resulting in a final concentration of 0.1-0.3% (w/v), preferably 0.2% (w/v) phosphoric acid, and/or in a pH of 4.0 ± 0.5,
(f) adjusting the pH in the product of step (e) to pH 4 or less, preferably pH 3.5-4,
(g) filtering or centrifuging the product of step (f),
(h) collecting the precipitate of step (g),
(i) treating and redissolving the precipitate of step (h) with a buffer to raise the pH to a physiological value, and
(j) repeating steps (d), (e), (f), (g), (h), and (i) at least once, preferably at least 3 times.

in an especially preferred way to perform this process,
(i) The centrifugation in step (b) is at a rotational speed of at least 3000 rpm for at least 5 min; and/or
(ii) a 20% (w/v) solution of metaphosphoric acid is used in step (e); and/or
(iii) an aqueous solution of hydrochloric acid, preferably concentrated hydrochloric acid, most preferably 30% (w/w) HCl is used in step (f) and the precipitation time in this step does not exceed 3 h; and/or
(iv) the centrifugation of step (g) is at 2000 rpm for 15 min; and/or
(v) the buffer in step (i) is phosphate buffered saline (PBS); and/or
(vi) in step (j) a 20% (w/v) solution of metaphosphoric acid is used instead of hydrochloric acid to adjust the pH and the centrifugation time is 10 min instead of 15 min.

Most favorable is a process wherein all features (i)-(vi) mentioned above are present.

Another specific anatoxin component of embodiment (1) is obtainable from cancer tissue instead of blood by a process comprising the following steps:
(a) treating a sample of cancer tissue either derived directly from a patient or prepared by *in vitro* tissue culture of cancer cells by standard textbook cell lysis methods and subsequent homogenization;
(b) centrifuging the homogenisate;
(c) -(j) as described above.

Preferably, the homogenization is done in a homogenizer at 60,000-70,000 rpm for 1 h.

The isolated product ("anatoxin component") of the process leading to the anatoxin component according to embodiment (1) can be used as vaccine in therapy and prophylaxis according to embodiment (1), (2) and (4).

A preferred embodiment (2) is a human anti cancer vaccine which in addition to the anatoxin component of embodiment (1) contains a lymphocyte component. This component is obtainable by two different alternative routes, both of which are equally apt: Firstly by treating vital lymphocytes of a person or a group of persons not suffering from cancer having the blood group 0, rhesus factor + ("healthy lymphocytes"), with DNA fragments prepared from vital lymphocytes of a patient or a group of patients suffering from cancer; secondly by treating said vital lymphocytes directly with vital lymphocytes of a person or a group of persons suffering from cancer ("cancer lymphocytes"). Both alternative aspects comprise a subsequent cultivation step after this treatment.

In a preferred aspect of embodiment (2) the lymphocyte component is obtained by a process com prising the following steps:
(a) isolation and purification of lymphocytes from a blood sample provided by one or several healthy persons;
(b) isolation and purification of lymphocytes from a blood sample provided by one or several cancer patients;
(c) treatment of healthy lymphocytes obtained in step (a) with either the vital cancer lymphocytes obtained in step (b) or with DNA fragments isolated from said vital cancer lymphocytes or isolated from tissue cultures of cancer cells;
(d) culturing the lymphocytes treated according to c) until no more lymphoblasts are detectable in the culture;
(e) collecting the resulting totipotent lymphocyte Tk specific cells; and
(f) using the collected cells either directly for preparation of the vaccine or after an optional final lysis step.

Thus, the lymphocyte component prepared with this process for use in embodiment (2) is either used directly after preparation of the vital totipotent lymphocyte Tk specific cells ("alive" lymphocyte component), or those cells are further submitted to a subsequent lysis step after their preparation ("non-alive" lymphocyte component). The lysis can be done by application of standard textbook methods, and is preferably done by ultrasonic shock. The resulting bi-hetero-vaccine of embodiment (2) for therapeutical application can thus be
A) "alive bi-hetero-vaccine" composed of the vital totipotent lymphocyte Tk specific cells and of the anatoxin; or
B) "non-alive bi-hetero-vaccine" composed of the isolation product after ultrasonic shock and of the anatoxin.

The weight percentage of the lymphocyte component in the bi-hetero-vaccine according to embodiment (2) is up to 70 %, preferably 30-60%, most preferably 50 %. An especially preferred aspect of embodiment (2) is that the same amount of the lymphocyte compound is combined with the anatoxin to prepare a vaccine, i.e. the rapport of anatoxin to lymphocyte compound is 50:50.

The lymphocytes needed for embodiment (2) of the invention are in one aspect of the invention isolated and purified as follows: a blood sample is taken and centrifuged. The supernatant is discarded, the sediment is re-dissolved in buffer, preferably PBS, and then submitted to a hypotonic shock, preferably by addition of 1.7% (w/v) aqueous NaCl. After erythrocyte lysis is finished, the sample is centrifuged and the sediment is washed several times with buffer. The lymphocytes thus purified can be used for preparation of the DNA fragments needed or for further selection of totipotent lymphocyte Tk specific cells.

Alternatively, the lymphocytes needed for embodiment (2) are in another aspect isolated and purified as follows: a blood sample is treated directly to effect erythrocyte lysis. Afterwards, the sample is treated in an identical way as above.

It is a prerequisite of embodiment (2) that the lymphocytes used are very pure.

An exemplified way to prepare the lymphocytes needed for the lymphocyte component for embodiment (2) of the invention is the following process comprising the following steps under sterile conditions:
(a) extracorporeally treating a defined amount of human blood of a patient suffering from cancer, who has not been treated by chemo- or radiotherapy, with a defined amount of heparin while shaking,
(b) diluting the product of step (a) containing vital lymphocytes with a defined amount of 1.7% (w/v) NaCl while shaking for a maximum of 15 s (hypotonic shock),
(c) leaving the resulting mixture (b) quiet until erythrocyte lysis is complete,
(d) collecting the sediment of (c),
(e) rinsing said precipitated lymphocytes with a buffer (PBS) and again repeating step (d) at least once, preferably by centrifugation at a rotational speed of at least 300 rpm for at least 10 min,
(f) optionally checking vitality of the lymphocytes,
(g) centrifuging said lymphocytes of step (f) at a rotational speed of 400 rpm for at least 5 minutes,
(h) rinsing the precipitate with a buffer and again repeating step (g) at least once, preferably twice,
(i) in vitro cultivation of the isolated and purified lymphocytes until their use in preparation of the lymphocyte compound.

In one preferred alternative aspect of embodiment (2) the method may be continued directly after step (h) by the following additional steps:
(i) storing a defined amount of human blood of a person having the blood group 0, rhesus factor + not suffering from cancer at a temperature of at least 37°C for at least 60 h,
(j) separating the lymphocytes from the product of step (i), preferably by the method of steps (a) to (h);
(k) preparing the DNA from a sample of cancerous lymphocytes prepared by steps (a) to (h) and cutting it into fragments by standard DNA fragmentation methods, preferably by endonuclease restriction, most preferably by use of a mixture of BamH I and BamH III according to manufacturer's instructions,
(l) mixing the same amounts of the product of step (j) and step (k),
(m) cultivating the mixture of step (I) at a temperature of at least 37 °C for at least 60 h, preferably at least 72 h,
(n) centrifugation of the culture and removal of the supernatant;
(o) redissolving the sediment in buffer;
(p) repeating steps (n) and (o) at least once, preferably twice.

In one preferred alternative aspect of embodiment (2) the method may be continued from step (n) by the following additional steps:
(n) treating the product of step (m) in an ultra sonificator,
(o) centrifuging the product of step (n) at a rotational speed of at least 1000 rpm for at least 5 min,
(p) removing the precipitate,
(q) treating the supernatant with an aqueous solution of hydrochloric acid and thereby lowering the pH to a value of 3,5 to 4,
(r) centrifuging the product of step (g) at a rotational speed of at least 1000 rpm for at least 10 min,
(s) treating and solving the precipitate of step (r) with a buffer to raise the pH to a physiological value and
(t) repeating steps (r) and (s) at least once to obtain the desired lymphocyte component of the vaccine.

The cells taken from the patients may in a third alternative aspect of lymphocyte preparation according to embodiment (2) be extracorporeally manipulated in the laboratory by isolating the lymphocytes of the cancer cells to prepare cultures for the breeding of the cells.

The time necessary for such breeding is about 72 h to 4 weeks and thus, the cells are stored in a fertile environment. Afterwards they may be used directly; no lysis step is necessary to remove erythrocytes.

In summary, the lymphocyte component of embodiment (2) is biochemically manipulated to obtain a hyper immunized lymphocyte component ("totipotent lymphocyte Tk specific cells") or its lysis products.

In a preferred aspect of embodiment (2), the totipotent lymphocyte Tk specific cells are used as cell suspension in culture broth with approximately 2 million cells/ml for preparation of the vaccine.

Preferably, all process steps leading to the vaccine of embodiment (1) or (2) should be done under sterile conditions.

After several years of research and development with several patients the inventor has come to the conclusion that the above referenced use according to embodiment (4) leads to extraordinary results, i.e. significant reduction of tumor size and very often a complete healing of patients suffering from different kinds of tumors.

The medicament according to embodiment (4) may be used for all kinds of cancer in a patient, including lung cancer, breast cancer, skin cancer, liver cancer, sarcoma, leukemia, bone cancer and Hodgkin-lymphoma. In particular the medicament may be used for therapy and prophylaxis of lung cancer, breast cancer, skin cancer, liver cancer and Hodgkin-lymphoma.

Once the vaccine has been prepared it is to be administered to the patient. If serum or cells from the patient were used to prepare the vaccine and the patient had a blood group different from 0+, it can be used just for vaccination of this patient or patients having the same blood group as the patient. If serum and cells were used which originated from one or several patients with exclusively blood group 0+, the vaccine may be injected to any patient suffering from cancer.

Whereas in the first case of manipulation and administration to a patient optimal results are received without contra-indication with respect to the blood group, the second variation of the process of the preparation of the vaccine requires the measurement of blood compatibility.

The application to the patient for the use according to embodiment (4) is in one preferred aspect done by intravenous or subcutaneous injection.

The "alive bi-hetero-vaccine" is preferably applied in the following way: One dose is 5 ml and incorporates 10 millions of alive cells plus anatoxic agents. In the beginning, the patient gets 8 doses with an interval time of 10 days, after 6 months 2 doses of 5 ml each every 15 days. The re-vaccination should be done with 3 doses of 5 ml each every 15 days one year after the 2^{nd} semi-annual dose.

The "non-alive bi-hetero-vaccine" is preferably applied in the following way: One dose is 1-2 ml, in very urgent cases preferably 2 ml, and incorporates 1 -2 million of alive cells or the corresponding amount of lysis products of 1-2 million totipotent lymphocyte Tk specific cells plus anatoxin. In the beginning, the patient is to be administered 1 dose/month with an interval time of 1 month, and at least 3 doses in total. 6 months after the first treatment said patient is to be treated again with 1-2 ml of vaccine. One year after the first treatment the patient is to be treated again with two doses of 1 ml within one month.

If rapid intervention is needed, the "alive bi-hetero-vaccine" should preferably be used.

Most preferably, the "non-alive" vaccines according to both major embodiments (1) and (2) are to be injected subcutaneous in an amount of 1 to 2 ml depending on the development of the desease. The vaccine is to be administered three times within 28 days. Six months after the first treatment the treatment is to be repeated with the same dosage each with 1 to 2 ml for enhancing the development of gene immunity. Again, one year after the first treatment the treatment is to be repeated by applying the vaccine two times within 28 days. At the end of the procedure the patient is completely healed.

After the administration of the second injection of the vaccine, one observes a reduction of the malicious tumor whereas during the following treatment one observes the total disappearance of the tumor mass (Example 3).

For the use of the vaccine according to embodiment (4), one alternatively preferred way of application is the combination of the vaccine according to embodiment (1) or (2) with a pharmaceutically suitable carrier, preferably with an aluminum phosphate gel, in order to allow the uptake of the vaccine in the body in a retarded form.

The invention is further explained and illustrated by the following examples.

### Examples

### Example 1 : Preparation of anatoxin

50 ml blood are taken from a patient suffering from cancer for the preparation of blood platelets. It has to be ensured that said patient has not been treated by radio- or chemo-therapy in advance.

25 ml of said blood are coagulated by a standard erythrocyte sedimentation rate procedure. From this blood the serum is taken and centrifuged for at least 1 min, preferably at least 5 min, at a rotational speed of at least 3000 rpm. The supernatant is treated with an aqueous solution of formaldehyde having a concentration of 37% (w/w) formaldehyde in an amount of 4 ml/l serum. Said reaction mixture is held at a temperature of 38°C for 28 days allowing the preparation of an anatoxin. Said anatoxin is then treated with an aqueous solution of phosphoric acid (m-HPO₃) (20% by weight) in an amount of 10 ml/l per I anatoxin.

If biological material from more than one patient is used, tissue cells from different patients with various forms of cancer and with a common blood group, preferably 0+ are cultivated *in vitro* by standard cultivation methods, preferably in a culture medium containing 83% Eagle MEM, 15% human inactive serum, 0.25% water soluble Trypsin (if cells are trypsinized before cultivation), 1% (v/v) of 20% (w/v) Kanamycin-solution.

4 ml of 37% formalin are added per I of supplied material. Blood serum from cancer patients is treated in the same way. The mixture is incubated at 38°C for 28 days after addition of the formalin and then stabilized by addition of 10 ml/l 20% (w/w) metaphosphoric acid.

By addition of an aqueous solution of hydrochloric acid (30 % by weight) the pH value is then adjusted to 3.5 to 4. Precipitation time should not exceed 3 h. Said anatoxin again is centrifuged for 15 m in at a rotational speed of 2000 rpm.

The precipitate is dissolved in a buffer (PBS; 8.0 % (w/v) NaCl, 0.2 % (w/v) KH₂PO₄, 2.9 % (w/v) Na₂HPO₄, 0.2 % (w/v) KCI) to adjust the pH at around 7 to 7.4. In order to purify the anatoxin, the solution again is treated with metaphosphoric acid (20 % by weight) lowering the pH to about 4.0. The mixture is centrifuged at 2000 rpm for 10 min, the sediment is diluted with PBS until the pH reaches 7-7.4.

Said procedure is repeated 2 to 3 times yielding in the immunogen of said anatoxin with a high immunogenic potential. The final sediment containing purified anatoxin is taken up in PBS pH 7.4 in a 1 :3 ratio and can then be stored or used as vaccine (alone or in combination with the lymphocyte component).

For preservation of this immunogen preserving agents, especially aluminum phosphate gel may be added. Their effect is the maintaining of the vaccine in a tissue pocket when it is injected, resulting in a slow diffusion of the vaccine into the organism.

### Example 2: Preparation of the lymphocyte component

Blood of healthy persons (group 1) and patients suffering from different forms of cancer (group 2) with the same blood group (0+) is separately for each blood sample exposed to a hypotonic shock by adding 1.7 % (w/v) NaCl in a ratio of 1 part blood, 5 parts NaCl-solution, and gently shaking the mixture for 15 s. Alternatively, 25 ml blood is treated with 10 units standard Heparin per 1 ml blood. Said blood can be stored in a container at a temperature of liquid nitrogen for several months and taken when necessary. One part of said blood containing vital lymphocytes is diluted with 5 parts of distilled water. During the following period of 15 s said blood is shaken vigorously. Immediately thereafter said liquid is added to 125 ml of a 1.7 % solution of NaCl.

After the shaking, the mixture is left quiet for 15 min.

Then the supernatant is removed and the sediment taken; alternatively, the complete reaction mixture is centrifuged for 20 min at a rotational speed of 400 rpm. At the end of said step a precipitate containing vital lymphocytes having a homogeneous color (silver) has been produced. It is necessary to check the vitality of said lymphocytes by routine determ ination.

The supernatant is removed and the precipitate is rinsed with a buffer (PBS) in a ratio of 1 part sediment/3 parts buffer and centrifuged for 10 m in at a rotational speed of 300 rpm. Said rinsing procedure is repeated 2 to 3 times with centrifugation times of 5 min.

The vitality of the cells in the precipitate is checked by adding an aqueous solution of 1 % of trypan blue. The observation with a microscope reveals colored lymphocytes being dead and uncolored vital lymphocytes.

From the precipitate, which comprises leukocytes, the lymphocytes are separated. In healthy persons, lymphocytes are normally ca. 30% of the leukocytes, but in cancerous patients, especially patients suffering leukemia and lymphoid cancer forms, the percentage of lymphocytes reaches 80-90 %, in case of lymphoblastic cancer forms even 90-95%. In these latter cases, it is not necessary to separate the lymphocytes from other leukocyte cells and cell com ponents.

It is self-evident that the complete procedure should be handled under sterile conditions.

Thus 2 lymphocyte groups result, one supplied by healthy individuals, the other by cancer patients.

For material multiplication, lymphocytes or cells from cancer affected organs can be cultivated in alimentary medium:
Lymphocyte cultivation medium contains 83% Eagle MEM, 15% human inactive serum, 0.01 per 1 ml Phythemagglutinin, 40 units/ml Heparin, 1% (v/v) of 20% (w/v) Kanamycin-solution; Tissue cell cultivation medium contains 83% Eagle MEM, 15% human inactive serum, 0.25% water soluble Trypsin (if cells are trypsinized before cultivation), 1% (v/v) of 20% (w/v) Kanamycin-solution.

The amount of lymphocytes to be filled in a container for incubation should not exceed 2 ml. The reaction mixture containing the cells and medium is held for 72 hours at a temperature of 37 °C by moving the reaction vessel approximately 30 times per hour. For breeding a standard culture medium is used. In particular, Eagle (MEM) containing serum human inactive 15 %, Phythemagglutinin 0.01 ml, Kanamycin 0.2% (w/v) and heparin (40 units) for 1 ml.

Thereafter, the reaction product is centrifuged for 1 min at a rotational speed of 500 rpm; alternatively, the lymphocyte mixture is centrifuged 15 min at 400 rpm. The supernatant is removed and stored at -85°C (only in case of supernatant of cancer patient lymphocytes) and the precipitate is rinsed with a buffer (PBS) in a ratio of precipitate:buffer 1:3. Again the rinsed precipitate is centrifuged for at least 5-10 min at a rotational speed of 400-600 rpm. The procedure is repeated twice.

The isolated lymphocytes and cells are checked m icroscopically for their vitality as outlined above.

The isolated cells are treated in different ways:
A) DNA is isolated from the cultivated lymphoblasts derived from cancer patients or the cultivated cells from cancerous organ/tissues by standard textbook DNA isolation methods. The isolated DNA is treated with a method resulting in DNA fragmentation, preferably by using endonucleases or other restriction enzymes. Most preferred is the use of BamH I and BamH III in combination according to manufacturer's instructions. The DNA fragments are then placed into the lymphocytes isolated from healthy persons ("healthy lymphocytes") by way of micromanipulation, electroporation, lipofectamin aided transfection or calcium phosphate mediated transfection. Preferred ways are micromanipulation and calcium phosphate mediated transfection. In the latter case, either a standard textbook method is used as described, e.g., in Frank, M. B. (ed.) Molecular Biology Protocols (1997, rev. 2000)), or DNA segments are added to a culture of healthy lymphocytes directly together with 0.2-1% (w/v) CaPO₄, preferably 0.5% (w/v) CaPO₄ ("calcium phosphate mediated diffusion").
B) Alternatively, lymphocytes derived from a healthy individual and those derived from a cancer patient are mixed in a 50:50 ratio.
   In this case, half of the isolated cell material is stored in a container under nitrogen atmosphere to allow a later reproduction. The other part of the reaction product is combined with the same amount of lymphocytes from a patient having the blood group 0, rhesus + who was diagnosed to be totally healthy. Said mixture was stored at 37 °C for 72 h observing a change in the morphology of the lymphocytes. In case (A), it is microscopically noticed that a great quantity of healthy lymphocytes is transformed into lymphoblastic ones during the incubation. In case (B), a numeric diminution of healthy lymphocyte cells and an increase of lymphoblastic ones is noted as well, but only after numerous recultivations taking a long time.

The content of the mixture is re-cultivated according to the method outlined above until the healthy lymphocytes are no longer transformed into lymphoblastic cells. Alternatively, the lymphocytes that do remain healthy are carefully divided from the other cells. The resulting cells are denominated "totipotent lymphocyte Tk specific cells". They should be able to bind to cancerous antigens and to lysis cells bearing those antigens.

The totipotent lymphocyte Tk specific cells are cultivated as outlined above. They are able to completely exterminate all lymphoblastic cells in cultures of tissue and lymphocyte cancer cells in vitro if they are added in a ratio of 2 parts totipotent lymphocyte Tk specific cells to 4 parts cancerous cells and incubated as outlined above.

The treatment of the healthy lymphocytes may also be performed with metabolic products of lym phoblastic cells or organic cancerous tissue.

The totipotent lymphocyte Tk specific cells are used as cell suspension with approximately 2 million cells/ml for preparation of the vaccine.

The reaction mixture is stored for 72 h in a thermostat at 37 °C.
It may be used directly for vaccine preparation or may be treated in an ultra sonificator. In the latter case, the reaction product is centrifuged to remove the destroyed mass of the cells. The supernatant is taken by withdrawing the precipitate. The supernatant is dissolved in an aqueous solution of hydrochloric acid (37%) adjusting the pH to 3.5 to 4. Again the reaction product is centrifuged for 10 min at a rotational speed of 1500 rpm.

The above referenced removed precipitate is dissolved in a buffer (PBS 3 ml per 1 ml of the mass of the precipitate). Again, said precipitate is centrifuged at a rotational speed of 400 rpm for 1 min. Said procedure is repeated 2 to 3 times yielding a hyper immune antigen.

Said antigen could be used for diagnostic purposes and as vaccine in therapy. In this respect, it is preferred to combine said antigen with an aluminum phosphate gel allowing the uptake of the vaccine in the body in a retarded form.

### Example 3: Therapeutical Application

For the treatment of patients suffering from cancer two types of vaccines have been prepared:
a) auto-vaccine which was injected to one person which had been the source of the serum used to produce the anatoxin;
b) hetero-vaccine which was prepared using the pooled serum taken from several persons. Thus, it could be used for all types of cancer and not only for one specific person, but for any patient.

### Medical Treatment of a patient with vaccine:

The "alive bi-vaccine" can be applied to a patient intravenous or subcutaneo us. One dose is 5 ml and incorporates 10 millions of alive cells plus anatoxic agents.

In the beginning, the patient gets 8 doses with an interval time of 10 days, after 6 months 2 doses of 5 ml each every 15 days. The re-vaccination should be done with 3 doses of 5 ml each every 15 days one year after the 2^{nd} semi-annual dose.

The dosage of the "non-alive bi-vaccine" is as follows: Three doses of 1-2 ml vaccine were given to the patients by subcutaneous injection each month. Accordingly, the treatment of the patients took at least 3 months. 6 months after the first treatment said patients were again treated with 1-2 ml vaccine. One year after the first treatment, the patients were again treated with 2 doses of 1 ml within 1 month.

Patients suffering *inter alia* from advanced state lung cancer, morbus Hodgkin, metastatical cancer originating from breast cancer, liver cancer, skin cancer showed significant reduction of tumor size up to complete destruction of the tumor after this treatment. Some patients which were initially diagnosed as incurable sowed complete remission after the treatment and lived on afterwards for 10 years and more without anew cancer illness.

## Claims

1. A human anti cancer vaccine comprising an anatoxin component which is obtainable by modifying in vitro blood serum or the supernatant of homogenized cancer tissue cells of a patient suffering from cancer, or the combined blood serum or supernatant of homogenized cancer tissue cells of a group of patients with blood serotype O⁺ suffering from cancer, with protein crosslinking agents.

2. The vaccine of claim 1, wherein
(i) the patient(s) has (have) not been treated by chemo- or radiotherapy before; and/or
(ii) the group of patients suffer from the same or different forms of cancer; and/or
(iii) the crosslinking agent is formaldehyde, preferably the final concentration range of formaldehyde during the treatment is 0.05-0.5 % by weight; and/or
(iv) the incubation time is 25-35 days, preferably at least 28 days; and/or
(v) the incubation takes place at a temperature of 38 ± 2°C, preferably 38 - 39°C.

3. The vaccine according to claim 1 or 2 wherein the anatoxin component is obtainable by a process comprising the following steps:
(a) extracorporeally coagulating blood of patient(s) suffering from cancer or homogenizing cancer tissue cells,
(b) centrifuging the product of step (a),
(c) combining the supernatant and/or serum with an aqueous solution of formaldehyde,
(d) incubating the mixture of step (c) at a temperature of 38°C +/- 2°C,
(e) treating the product of step (d) with an aqueous solution of phosphoric acid, preferably resulting in a final concentration of 0.1-0.3% (w/v), preferably 0.2% (w/v) phosphoric acid,
(f) adjusting the pH in the product of step (e) to pH 4 or less, preferably pH 3.5-4,
(g) filtering or centrifuging the product of step (f),
(h) collecting the precipitate of step (g),
(i) treating and redissolving the precipitate of step (h) with a buffer to raise the pH to a physiological value, and
(j) repeating steps (d), (e), (f), (g), (h), and (i) at least once, preferably at least 3 times.

4. The vaccine of claim 3, wherein
(i) a 20% (w/v) solution of metaphosphoric acid is used in step (e); and/or
(ii) an aqueous solution of hydrochloric acid, preferably concentrated hydrochloric acid, most preferably 30% (w/w) HCl is used in step (f) and the precipitation time in this step does not exceed 3 h; and/or
(iii) the buffer in step (i) is phosphate buffered saline (PBS).

5. The vaccine of anyone of claims 1 to 4, which additionally comprises up to 70% (w/w), preferably 30-60% (w/w), most preferably 50% (w/w), of a lymphocyte component obtainable by contacting vital lymphocytes of one or several person(s) not suffering from cancer ("healthy lymphocytes") with vital lymphocytes of one or several patient(s) suffering from cancer ("cancer lymphocytes") or with DNA fragments isolated from cancer lymphocytes.

6. The vaccine of claim 5, wherein the lymphocyte component is obtainable by a process comprising the following steps:
(a) isolating and purifying of healthy lymphocytes from a blood sample provided by one or several healthy persons;
(b) isolating and purifying of cancer lymphocytes from a blood sample provided by one or several cancer patients having the same or different forms of cancer;
(c) treating healthy lymphocytes obtained in step (a) with either the vital cancer lymphocytes obtained in step (b) or with DNA fragments isolated from said vital cancer lymphocytes;
(d) culturing the lymphocytes treated in step (c) until no more lymphoblasts are detectable in the culture;
(e) collecting the resulting cells ("totipotent lymphocyte Tk specific cells").

7. The vaccine of claim 6, wherein
(i) the patient(s) of step (b) has (have) not been treated by chemo- or radiotherapy before; and/or
(ii) the cancer lymphocyte treatment of step (c) comprises mixing of purified healthy and cancer lymphocytes in a 50:50 ratio and incubation at 37°C for 72 h; and/or
(iii) the DNA fragment treatment of step (c) comprises micromanipulation, electroporation, lipofectamin aided transfection or calcium phosphate mediated diffusion; and/or
(iv) the culturing step (d) comprises incubation at a temperature of at least 37°C.

8. The vaccine of claim 6 or 7, wherein
(i) the ratio of the anatoxin and the lymphocyte component is 50:50; and/or
(ii) the vaccine further comprises one or more stabilizing and/or retarding agents, most preferably an aluminum or phosphate silica gel; and/or
(iii) the vaccine is suitable for treatment of all kinds of cancer in a patient, including lung cancer, breast cancer, skin cancer, liver cancer, sarcoma, leukemia, bone cancer and Hodgkin-lymphoma.

9. Process for production of a human anticancer vaccine as defined in anyone of claims 1 to 8, preferably as defined in claims 5 to 8, which comprises admixing the anatoxin component with the lymphocyte component.

10. Use of a vaccine component as defined in anyone of claims 1 to 8 for preparing a medicament for treatment or prophylaxis of all kinds of cancer in a human patient, including lung cancer, breast cancer, skin cancer, liver cancer, sarcoma, leukemia, bone cancer and Hodgkin-lymphoma.

## Patentansprüche

1. Humaner Antikrebsimpfstoff, umfassend eine Anatoxinkomponente, die durch Modifizieren von Blutserum oder des Überstands von homogenisierten Krebsgewebezellen eines Patienten, der an Krebs leidet, oder des kombinierten Blutserums oder Überstands von homogenisierten Krebsgewebezellen einer Gruppe von Patienten der Blutgruppe 0+, die an Krebs leiden, in vitro mit Proteinvernetzungsmitteln erhältlich ist.

2. Impfstoff gemäß Anspruch 1, wobei
(i) der oder die Patienten zuvor nicht durch Chemo- oder Strahlentherapie behandelt wurde(n); und/oder
(ii) die Gruppe von Patienten an derselben oder an unterschiedlichen Krebsformen leiden; und/oder
(iii) es sich bei dem Vernetzungsmittel um Formaldehyd handelt, vorzugsweise der endgültige Konzentrationsbereich des Formaldehyds während der Behandlung 0,05 bis 0,5 Gew.-% beträgt; und/oder
(iv) die Inkubationszeit 25-35 Tage, vorzugsweise wenigstens 28 Tage, beträgt; und/oder
(v) die Inkubation bei einer Temperatur von 38 ± 2 °C, vorzugsweise 38-39 °C, stattfindet.

3. Impfstoff gemäß Anspruch 1 oder 2, wobei die Anatoxinkomponente durch ein Verfahren erhältlich ist, das die folgenden Schritte umfasst:
(a) extrakorporales Koagulieren von Blut eines oder mehrerer Patienten, der oder die an Krebs leiden, oder Homogenisieren von Krebsgewebezellen;
(b) Zentrifugieren des Produkts von Schritt (a);
(c) Kombinieren des Überstands und/oder des Serums mit einer wässrigen Lösung von Formaldehyd;
(d) Inkubieren des Gemischs von Schritt (c) bei einer Temperatur von 38°C+/-2°C;
(e) Behandeln des Produkts von Schritt (d) mit einer wässrigen Lösung von Phosphorsäure, was vorzugsweise zu einer Endkonzentration von 0,1-0,3% (w/v), vorzugsweise 0,2% (w/v), Phosphorsäure führt;
(f) Einstellen des pH-Werts im Produkt von Schritt (e) auf pH 4 oder weniger, vorzugsweise pH 3,5-4;
(g) Filtrieren oder Zentrifugieren des Produkts von Schritt (f);
(h) Gewinnen des Niederschlags von Schritt (g);
(i) Behandeln und Wiederauflösen des Niederschlags von Schritt (h) mit einem Puffer, um den pH-Wert auf einen physiologischen Wert anzuheben; und
(j) Wiederholen der Schritte (d), (e), (f), (g), (h) und (i) wenigstens einmal, vorzugsweise wenigstens dreimal.

4. Impfstoff gemäß Anspruch 3, wobei
(i) in Schritt (e) eine 20%-ige (w/v) Lösung von Metaphosphorsäure verwendet wird; und/oder
(ii) in Schritt (f) eine wässrige Lösung von Chlorwasserstoffsäure, vorzugsweise konzentrierte Salzsäure, am meisten bevorzugt 30%-ige (w/w) HCl, verwendet wird und die Fällungszeit in diesem Schritt 3 h nicht überschreitet; und/oder
(iii) es sich bei dem Puffer in Schritt (i) um phosphatgepufferte Kochsalzlösung (PBS) handelt.

5. Impfstoff gemäß einem der Ansprüche 1 bis 4, der zusätzlich bis zu 70% (w/w), vorzugsweise 30-60% (w/w), am meisten bevorzugt 50% (w/w), einer Lymphocytenkomponente umfasst, die dadurch erhältlich ist, dass man lebensfähige Lymphocyten von einer oder mehreren Personen, die nicht an Krebs leiden ("gesunde Lymphocyten"), mit lebensfähigen Lymphocyten von einem oder mehreren Patienten, die an Krebs leiden ("Krebslymphocyten"), oder mit DNA-Fragmenten, die aus Krebslymphocyten isoliert wurden, in Kontakt bringt.

6. Impfstoff gemäß Anspruch 5, wobei die Lymphocytenkomponente durch ein Verfahren erhältlich ist, das die folgenden Schritte umfasst:
(a) Isolieren und Reinigen von gesunden Lymphocyten aus einer Blutprobe, die von einer oder mehreren gesunden Personen stammt;
(b) Isolieren und Reinigen von Krebslymphocyten aus einer Blutprobe, die von einem oder mehreren Krebspatienten, die dieselbe oder unterschiedliche Krebsformen aufweisen, stammt;
(c) Behandeln von gesunden Lymphocyten, die in Schritt (a) erhalten wurden, mit entweder den in Schritt (b) erhaltenen lebensfähigen Krebslymphocyten oder mit DNA-Fragmenten, die aus den lebensfähigen Krebslymphocyten isoliert wurden;
(d) Kultivieren der in Schritt (c) behandelten Lymphocyten, bis in der Kultur keine Lymphoblasten mehr nachweisbar sind;
(e) Auffangen der resultierenden Zellen ("totipotente Lymphocyten-Tkspezifische Zellen").

7. Impfstoff gemäß Anspruch 6, wobei
(i) der oder die Patienten von Schritt (b) zuvor nicht durch Chemo- oder Strahlentherapie behandelt wurde(n); und/oder
(ii) die Krebslymphocyten-Behandlung von Schritt (c) das Mischen von gereinigten gesunden Lymphocyten und Krebslymphocyten in einem Verhältnis von 50:50 und Inkubation bei 37 °C während 72 h umfasst; und/oder
(iii) die DNA-Fragment-Behandlung von Schritt (c) eine Mikromanipulation, Elektroporation, Lipofectamin-gestützte Transfektion oder Calciumphosphat-vermittelte Diffusion umfasst; und/oder
(iv) der Schritt des Kultivierens (d) eine Inkubation bei einer Temperatur von wenigstens 37 °C umfasst.

8. Impfstoff gemäß Anspruch 6 oder 7, wobei
(i) das Verhältnis des Anatoxins und der Lymphocytenkomponente 50:50 beträgt; und/oder
(ii) der Impfstoff weiterhin einen oder mehrere Stabilisatoren und/oder Verzögerungsmittel, am meisten bevorzugt ein Aluminium- oder Phosphat-Silicagel, umfasst; und/oder
(iii) der Impfstoff für die Behandlung aller Arten von Krebs bei einem Patienten einschließlich Lungenkrebs, Brustkrebs, Hautkrebs, Leberkrebs, Sarkomen, Leukämie, Knochenkrebs und Hodgkin-Lymphom geeignet ist.

9. Verfahren zur Herstellung eines humanen Antikrebsimpfstoffs gemäß einem der Ansprüche 1 bis 8, vorzugsweise gemäß den Ansprüchen 5 bis 8, umfassend das Mischen der Anatoxinkomponente mit der Lymphocytenkomponente.

10. Verwendung einer Impfstoffkomponente gemäß einem der Ansprüche 1 bis 8 zur Herstellung eines Medikaments zur Behandlung oder Prophylaxe aller Arten von Krebs bei einem humanen Patienten einschließlich Lungenkrebs, Brustkrebs, Hautkrebs, Leberkrebs, Sarkomen, Leukämie, Knochenkrebs und Hodgkin-Lymphom.

## Revendications

1. Vaccin contre le cancer humain comprenant un composant d'anatoxine qui peut être obtenu par modification in vitro du sérum sanguin ou du surnageant de cellules de tissu cancéreux homogénéisées d'un patient souffrant d'un cancer, ou du sérum sanguin combiné ou du surnageant de cellules de tissu cancéreux homogénéisées combiné d'un groupe de patients ayant un sérotype sanguin O⁺ et souffrant d'un cancer, avec des agents de réticulation des protéines.

2. Vaccin selon la revendication 1, où
(i) le ou les patient(s) n'a ou n'ont pas été traité(s) par chimiothérapie ou radiothérapie auparavant; et/ou
(ii) le groupe de patients souffrent de la même forme ou différentes formes de cancer; et/ou
(iii) l'agent de réticulation est le formaldéhyde, de préférence, l'intervalle de concentration finale de formaldéhyde durant le traitement est de 0,05-0,5% en poids; et/ou
(iv) le temps d'incubation est de 25-35 jours, de préférence, d'au moins 28 jours; et/ou
(v) l'incubation a lieu à une température de 38 ± 2°C, de préférence, 38 - 39°C.

3. Vaccin selon la revendication 1 ou 2, dans lequel le composant d'anatoxine peut être obtenu par un procédé comprenant les étapes suivantes:
(a) coagulation extracorporelle du sang du ou des patient(s) souffrant d'un cancer ou homogénéisation des cellules de tissu cancéreux,
(b) centrifugation du produit de l'étape (a),
(c) combinaison du surnageant et/ou du sérum avec une solution aqueuse de formaldéhyde,
(d) incubation du mélange de l'étape (c) à une température de 38°C ± 2°C,
(e) traitement du produit de l'étape (d) avec une solution aqueuse d'acide phosphorique, de préférence, donnant lieu à une concentration finale de 0,1-0,3% (p/v), de préférence, de 0,2% (p/v) d'acide phosphorique,
(f) ajustement du pH du produit de l'étape (e) à pH 4 ou moins, de préférence, à pH 3,5-4,
(g) filtration ou centrifugation du produit de l'étape (f),
(h) collecte du précipité obtenu à l'étape (g),
(i) traitement et redissolution du précipité de l'étape (h) avec un tampon pour augmenter le pH à une valeur physiologique, et
(j) répétition des étapes (d), (e), (f), (g), (h) et (i) au moins une fois, de préférence, au moins 3 fois.

4. Vaccin selon la revendication 3, où
(i) une solution à 20% (p/v) d'acide métaphosphorique est utilisée dans l'étape (e); et/ou
(ii) une solution aqueuse d'acide chlorhydrique, de préférence, d'acide chlorhydrique concentré, le plus préférablement, de HCl à 30% (p/p), est utilisée dans l'étape (f) et le temps de précipitation dans cette étape ne dépasse pas 3 h; et/ou
(iii) le tampon de l'étape (i) est une solution tamponnée de phosphate (STP).

5. Vaccin selon l'une quelconque des revendications 1 à 4, qui comprend en outre jusqu'à 70% (p/p), de préférence, 30-60% (p/p), le plus préférablement, 50% (p/p), d'un composant lymphocytaire pouvant être obtenu par mise en contact des lymphocytes vivants d'une ou de plusieurs personne(s) ne souffrant pas de cancer ("lymphocytes sains") avec les lymphocytes vivants d'un ou de plusieurs patient(s) souffrant de cancer ("lymphocytes cancéreux") ou avec des fragments d'ADN isolés à partir de lymphocytes cancéreux.

6. Vaccin selon la revendication 5, où le composant lymphocytaire peut être obtenu par un procédé comprenant les étapes suivantes:
(a) isolement et purification des lymphocytes sains à partir d'un échantillon de sang prélevé chez une ou plusieurs personnes saines;
(b) isolement et purification des lymphocytes cancéreux à partir d'un échantillon de sang prélevé chez un ou plusieurs patients cancéreux ayant la même forme ou différentes formes de cancer;
(c) traitement des lymphocytes sains obtenus à l'étape (b) avec soit les lymphocytes cancéreux vivants obtenus à l'étape (b), soit les fragments d'ADN isolés à partir desdits lymphocytes cancéreux vivants;
(d) mise en culture des lymphocytes traités à l'étape (c) jusqu'à ce que l'on ne puisse plus détecter de lymphoblastes dans la culture;
(e) collecte des cellules résultantes ("cellules totipotentes spécifiques des lymphocytes Tk").

7. Vaccin selon la revendication 6, où
(i) le ou les patient(s) de l'étape (b) n'a ou n'ont pas été traité(s) par chimiothérapie ou radiothérapie auparavant; et/ou
(ii) le traitement des lymphocytes cancéreux de l'étape (c) comprend le mélange des lymphocytes sains et des lymphocytes cancéreux purifiés dans un rapport de 50:50 et une incubation à 37°C pendant 72 h; et/ou
(iii) le traitement des fragments d'ADN de l'étape (c) comprend une micromanipulation, une électroporation, une transfection avec l'aide de la lipofectamine ou une diffusion au phosphate de calcium; et/ou
(iv) l'étape de culture (d) comprend une incubation à une température d'au moins 37°C.

8. Vaccin selon la revendication 6 ou 7, où
(i) le rapport de l'anatoxine au composant lymphocytaire est de 50:50; et/ou
(ii) le vaccin comprend en outre un ou plusieurs agents stabilisants et/ou retardateurs, le plus préférablement, un gel de silice à l'aluminium ou au phosphate; et/ou
(iii) le vaccin est approprié pour le traitement de tout type de cancer chez un patient, y compris le cancer du poumon, le cancer du sein, le cancer de la peau, le cancer du foie, le sarcome, la leucémie, le cancer des os et le lymphome de Hodgkin.

9. Procédé de production d'un vaccin contre le cancer humain tel que défini dans l'une quelconque des revendications 1 à 8, de préférence, tel que défini dans les revendications 5 à 8, qui comprend le mélange du composant d'anatoxine ave le composant lymphocytaire.

10. Utilisation d'un composant de vaccin tel que défini dans l'une quelconque des revendications 1 à 8 pour la préparation d'un médicament destiné au traitement ou à la prophylaxie de tous les types de cancer chez un patient humain, y compris le cancer du poumon, le cancer du sein, le cancer de la peau, le cancer du foie, le sarcome, la leucémie, le cancer des os et le lymphome de Hodgkin.
